(19) Europäisches Patentamt
European Patent Office
Office européen des brevets

(11) Numéro de publication: **0 536 012 B1**

# (12) FASCICULE DE BREVET EUROPEEN

(45) Date de publication de fascicule du brevet: **11.01.95**

(51) Int. Cl.[6]: **C07C 37/00**, C07C 39/38

(21) Numéro de dépôt: **92402327.8**

(22) Date de dépôt: **24.08.92**

## (54) Procédé de débromation de dérivés dibromés du naphtol.

(30) Priorité: **04.10.91 FR 9112268**

(43) Date de publication de la demande:
**07.04.93 Bulletin 93/14**

(45) Mention de la délivrance du brevet:
**11.01.95 Bulletin 95/02**

(84) Etats contractants désignés:
**AT BE CH DE DK ES FR GB GR IE IT LI LU NL PT SE**

(56) Documents cités:
**EP-A- 0 179 447**
**US-A- 2 025 032**

(73) Titulaire: **POTASSE ET PRODUITS CHIMIQUES**
**95 rue du Général de Gaulle**
**F-68800 Thann (FR)**

(72) Inventeur: **Jacquot, Roland**
**93 Chemin de la Courtille**
**F-69110 Saint Foy les Lyon (FR)**

(74) Mandataire: **Collier, Jeremy Austin Grey et al**
**J.A.KEMP & CO.**
**14, South Square**
**Gray's Inn**
**London WC1R 5LX (GB)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 536 012 B1

Rank Xerox (UK) Business Services
(3.10/3.09/3.3.3)

## Description

La présente invention porte sur un procédé de débromation de dérivés dibromés du naphtol. Plus précisément, l'invention concerne un procédé de monodébromation de dérivés dibromés du naphtol de formule (1) :

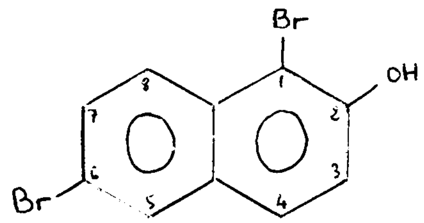

(1)

par hydrodébromation catalytique régio-sélective.

Les produits de réaction plus particulièrement visés par le procédé de débromation selon l'invention répondent à la formule (2) :

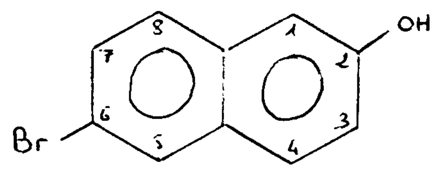

(2)

Le bromo-6 hydroxy-2 naphtalène (aussi appelé bromo-6 β naphtol) de formule (2) ci-dessus est un produit particulièrement intéressant et important. Il est en effet utilisé pour la synthèse du bromo-6 méthoxy-2 naphtalène (par une alkylation au moyen de sulfate de diméthyle ou de méthanol), ce dernier produit étant, quant à lui, largement utilisé pour obtenir le naproxène ou la nabumétone, qui sont notoirement connus pour leurs propriétés thérapeutiques anti-inflammatoires, ou bien encore le methallenestril, qui est un oestrogène (voir à ce sujet : the Merck Index, eleventh edition, 1989, pages 1002, 1014 et 937).

Selon l'enseignement du document EP-A-179 447, le bromo-6 hydroxy-2 naphtalène peut être préparé par réduction métallique stoëchiométrique du dibromo-1,6 hydroxy-2 naphtalène, selon la réaction suivante :

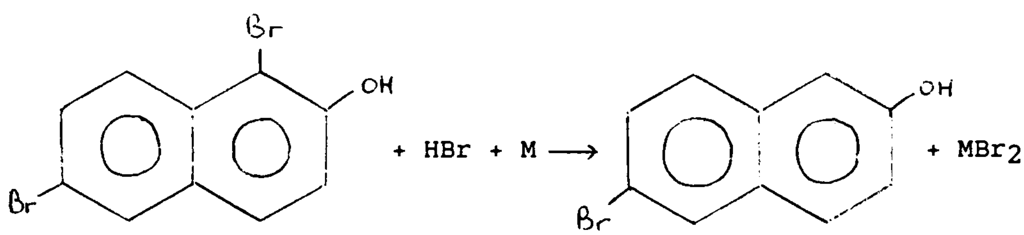


où M désigne un métal réducteur tel que fer ou étain, les dérivés dibromés ci-dessus pouvant eux-mêmes être préparés simplement par bromation directe du β naphtol :

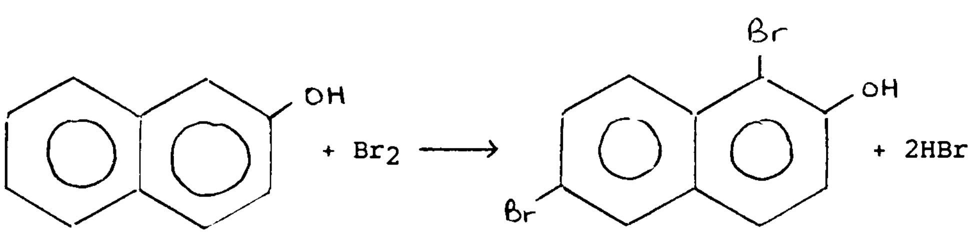

Toutefois, la réduction des dérivés dibromés du naphtol en dérivés monobromés selon un procédé tel que décrit ci-dessus présente le désavantage, entre autres, de nécessiter une consommation importante en métal, métal qui se retrouve par ailleurs sous la forme d'un effluent perdu, difficilement valorisable, et parfois polluant, comme par exemple Fe $Br_2$.

En outre, les rendements de réaction en dérivés monobromés désirés, par un tel procédé, peuvent s'avérer insuffisants.

La présente invention a pour but de proposer un procédé de débromation de certains dérivés dibromés du naphtol qui permette d'une part d'obvier aux inconvénients susmentionnés, et d'autre part de réaliser une débromation régio-sélective, en particulier en position 1, avec un rendement élevé.

A cet effet, il est maintenant proposé un nouveau procédé de débromation de dérivés dibromés du naphtol, ledit procédé étant caractérisé par le fait que l'on fait réagir, dans un solvant organique acide et en présence d'un catalyseur à base de carbure de tungstène, (i) un dérivé dibromé du naphtol de formule :

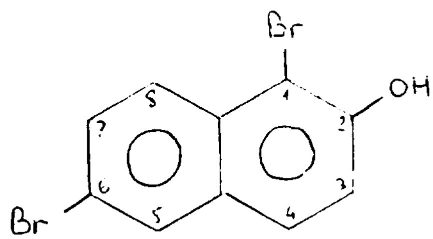

(1)

avec (ii) de l'hydrogène moléculaire ou un composé susceptible de générer, dans le milieu de réaction, de l'hydrogène naissant.

Selon un mode préféré de réalisation du procédé selon l'invention, la réaction est conduite en présence de fer et/ou de cuivre.

Le procédé selon l'invention présente de nombreux avantages et une grande souplesse d'utilisation. Il permet tout d'abord d'éviter la consommation stoëchiométrique de métaux réducteurs. Par ailleurs, de façon inattendue et surprenante, il est également hautement sélectif, en ce sens que, pour les produits de formule (1) ci-dessus, seul l'atome de brome en position 1 est substitué, et ceci même en cas d'utilisation d'un fort excès stoëchiométrique d'hydrogène. Les rendements en dérivés monobromés sont élevés.

La réaction peut en outre être conduite dans une large gamme de pressions et de températures, et selon de nombreux modes de réalisation.

Elle peut ainsi être réalisée en batch, en semicontinu, en continu, en réacteur agité, ou en lit fixe ruisselant.

Dans tous les cas, il y a récupération et réutilisation du catalyseur, ce qui ajoute à l'économie du procédé. Compte tenu du fait que l'on travaille dans les conditions d'une catalyse hétérogène, la récupération ultérieure du catalyseur est très facile puisque pouvant être effectuée par des moyens simples, comme une filtration ou une décantation.

Enfin, le procédé selon l'invention présente l'avantage de pouvoir être conduit directement, sans séparation ou purification préalable, sur le produit de réaction obtenu par bromation directe du $\beta$ naphtol.

Mais d'autres caractéristiques, aspects et avantages de l'invention apparaîtront encore plus clairement à la lecture de la description qui va suivre, et des exemples concrets mais non limitatifs destinés à l'illustrer.

Les catalyseurs utilisés dans la présente invention sont des catalyseurs à base de carbure de tungstène. Ces catalyseurs peuvent également comprendre, en plus du carbure de tungstène, un autre ou d'autres monocarbures de métal. En tant qu'autres monocarbures de métal, on peut notamment citer les carbures de molybdène, de vanadium, de niobium, de tantale, de titane, de fer et de chrome ; ceux-ci sont largement décrits dans la littérature. La quantité de ces autres carbures de métal représente, de préférence, de 0,01 à 50 % en poids par rapport à la totalité des carbures présents.

Bien que le carbure de tungstène soit déjà un produit bien connu en soi, on notera que son utilisation comme catalyseur d'hydrodébromation selon un procédé conforme à l'invention est, quant à elle, tout à fait nouvelle.

Le catalyseur est soit à base de carbone de tungstène massique, soit à base de carbure de tungstène supporté. Comme support, on peut notamment utiliser les oxydes, tels que la silice, l'alumine et le dioxyde de titane, ou du charbon.

Ainsi, le catalyseur peut se présenter sous la forme d'un substrat monolithique (nid d'abeilles ou autres) en carbure de tungstène, ou d un substrat monolithique revêtu d'une couche en carbure de tungstène, ou bien encore se présenter sous la forme de produits divisés en, ou revêtus de, carbure de tungstène. Par forme divisée, on entend des produits pulvérulents (poudres) et également les articles obtenus par mise en forme de ces produits (billes, pastilles, boulettes, granulés, extrudés, agglomérés, et autres, de section circulaire, ovale, trilobée ou multilobée, pleine ou creuse).

Les catalyseurs de type billes, pastilles et autres, présentent l'avantage de pouvoir être séparés ultérieurement du milieu de réaction très rapidement par simple décantation. Les catalyseurs de type pulvérulent nécessitent généralement, pour leur séparation, une étape de filtration.

Bien entendu, tous les catalyseurs précités sont choisis avec une surface spécifique convenant pour l'application considérée. Dans la pratique, on peut mettre en oeuvre un carbure de tungstène dont la surface spécifique (BET) varie de 0,1 à plusieurs centaines de $m^2/g$, et en particulier de 1 à 300 ou 400 $m^2/g$.

A cet effet, on pourra utiliser soit des carbures de tungstène disponibles dans le commerce soit des carbures de tungstène que l'on aura synthétisés selon tout procédé connu en soi. A titre d'exemples, des carbures de tungstène hautes surfaces spécifiques peuvent être fabriqués selon le procédé décrit dans la demande de brevet PCT/FR 90/00204.

La quantité de catalyseur à utiliser n'est pas critique et peut varier dans de larges limites ; généralement on utilise de 0,01 % à 50 % en poids de catalyseur par rapport à la quantité de dérivé dibromé engagée.

Selon l'invention, il est également possible d'introduire dans le milieu de réaction de faibles quantités de cuivre et/ou de fer, et ceci dans le but d'améliorer sensiblement les rendements en dérivé monobromé désiré. Ces éléments peuvent être apportés à l'état métallique ou sous la forme d'un sel soluble dans le milieu de réaction. La quantité, en mole, de fer et/ou de cuivre que l'on met généralement en oeuvre est alors comprise entre 0,01 et 0,1 fois la quantité en mole de dérivé dibromé engagée dans la réaction. On notera que de telles quantités sont considérablement plus faibles que les quantités stoëchiométriques qu'il est nécessaire d'utiliser conformément au procédé du document EP-A-179 447 précité. A l'inverse du catalyseur à base de carbure de tungstène, le fer et/ou cuivre engagé est consommé lors de la réaction.

Selon l'invention, la réaction doit être conduite en milieu solvant.

Il a été trouvé que le choix du solvant à utiliser revêtait une importance particulière, et que ce choix devait se limiter à des solvants organiques, et plus particulièrement à des solvants organiques acides.

Selon l'invention, on doit entendre par solvants organiques acides :

- soit des solvants organiques protiques choisis parmi les acides carboxyliques, simples ou fonctionnalisés ;
- soit des solvants organiques aprotiques contenant au moins un acide organique ou inorganique.

En particulier, il a été trouvé que les solvants organiques protiques de type alcoolique ne convenaient pas pour la présente invention.

Les solvants organiques préférés pour la mise en oeuvre du procédé selon l'invention sont les acides carboxyliques, simples ou fonctionnalisés, les hydrocarbures aromatiques et les hydrocarbures halogénés, les éthers et les esters.

A titre d'exemples non limitatifs d'acides carboxyliques convenant comme solvant pour la présente invention, on peut citer l'acide méthanoïque, éthanoïque, propanoïque, butanoïque, et trifluoroacétique. Par acides carboxyliques, on entend bien entendu couvrir également les acides polycarboxyliques, simples ou fonctionnalisés, qui conviennent ici tout à fait lorsqu'ils sont liquides dans les conditions de la réaction.

A titre d'exemples non limitatifs de solvants organiques aprotiques préférés convenables, on peut citer entre autres :

- parmi les hydrocarbures aromatiques : le benzène et les alkylbenzènes (éthyl-, butyl-, propylbenzène...), le toluène et les xylènes ;
- parmi les hydrocarbures halogénés, en particulier fluorés et chlorés, de composés paraffiniques, cycloparaffiniques, et aromatiques : le dichlorométhane, le dichloro-1,2 éthane et le chlorobenzène ;
- les éthers, tels que notamment l'éther d'isopropyle ;
- les esters, tels que notamment les acétates et les benzates, en particulier d'alkyles, comme par exemple l'acétate d'éthyle ou le benzoate de méthyle.

Bien entendu, il est également tout à fait possible, dans le cadre de la présente invention, d'utiliser à titre de solvant soit des mélanges d'acides carboxyliques, soit des mélanges de solvants organiques aprotiques, soit des mélanges entre des acides carboxyliques et des solvants organiques aprotiques.

A titre d'exemples non limitatifs d'acides pouvant être contenus, seuls ou en mélanges, dans les solvants organiques aprotiques tels que susmentionnés, on peut citer :

- parmi les acides inorganiques : l'acide phosphorique, l'acide sulfurique, les acides halogéniques, tels que l'acide chlorhydrique ou l'acide bromhydrique ;
- parmi les acides organiques : les acides carboxyliques susmentionnés, l'acide méthanesulfonique, triflique, éthanesulfonique ou benzènesulfonique, oxalique, malonique, succinique, glutarique, adipique, maléique, fumarique, phtalique et méllitique.

La quantité d'acide, en mole, qui est contenue dans le solvant organique aprotique est généralement comprise entre 0,1 et 5 fois la quantité en mole de dérivés dibromés engagée, de préférence entre 0,1 et 2 fois cette quantité.

La quantité d'hydrogène à utiliser peut varier dans de larges limites ; elle doit néanmoins correspondre au minimum à la quantité stoëchiométrique nécessaire pour permettre la substitution complète de la moitié des atomes de brome qui ont été apportés sous la forme du composé dibromé initial. Il n'y a pas de quantité limite supérieure à respecter.

Selon l'invention, l'hydrogène est de préférence utilisé sous une forme gazeuse moléculaire ($H_2$). On peut néanmoins également utiliser de l'hydrogène naissant, c'est à dire de l'hydrogène formé in situ dans le milieu de réaction par décomposition d'un composé précurseur tel qu'un formiate ou l'acide formique.

Les températures mises en oeuvre pour conduire la réaction peuvent varier dans de très larges limites.

On peut ainsi opérer de la température ambiante jusqu'à, théoriquement, la température d'ébullition du solvant utilisé, en veillant toutefois à ne pas excéder des températures où le dérivé dibromé et/ou le produit de réaction pourraient se décomposer ; dans la pratique, on travaille généralement à des températures comprises entre 20 °C et 200 °C, et de préférence entre 50 et 150 °C.

La réaction peut être conduite soit à pression atmosphérique dans un réacteur type ouvert, ou lit fixe ruisselant, dans lequel on fait barboter un courant continu d'hydrogène, soit, de préférence, sous pression autogène dans un réacteur fermé, du type autoclave, contenant une atmosphère d'hydrogène. Dans ce dernier cas, la pression en hydrogène peut aller de 1 à 50 bars, et de préférence de 5 à 20 bars.

La réaction est de préférence conduite sous agitation, et ceci généralement jusqu'à disparition complète ou quasi-complète du dérivé dibromé du naphtol introduit comme réactif.

En fin de réaction, on sépare du milieu réactionnel le dérivé monobromé obtenu, et ceci par tout moyen connu en soi, tel que par exemple filtration, décantation, centrifugation, extraction ou distillation.

Ainsi, par exemple, on pourra procéder tout d'abord à la récupération des catalyseurs, notamment par filtration ou décantation, puis à la séparation du dérivé monobromé et de la phase solvante organique, par exemple par extraction à l'eau ou distillation.

Les catalyseurs et/ou les solvants ainsi récupérés, après éventuellement purification, peuvent alors être recyclés en tête du procédé.

Le dérivé monobromé récupéré peut, quant à lui, subir des étapes complémentaires de purification, si nécessaire.

Des exemples illustrant l'invention vont maintenant être donnés.

Dans ces exemples, TT désigne le Taux de Transformation, c'est à dire le rapport :

$$\frac{\text{quantité en mole de dérivé dibromé transformée}}{\text{quantité en mole de dérivé dibromé introduite}} \times 100$$

RR désigne le Rendement de la Réaction vis à vis d'un produit de réaction donné, c'est à dire le rapport :

$$\frac{\text{quantité en mole d'un produit formé}}{\text{quantité en mole de dérivé dibromé introduite}} \times 100$$

RT traduit la sélectivité de la réaction pour un produit de réaction donné, il est défini par le rapport RR/TT.

**EXEMPLE 1** : utilisation d'un catalyseur WC seul

Dans une ampoule en verre de 35 ml, on introduit : 1,2 g de dibromo-1,6 naphtol-2 ; 0,47 g d'une poudre de carbure de tungstène WC (granulométrie moyenne : 1 µm ; surface spécifique : 1,6 $m^2/g$ ) ; et 15 ml d'acide acétique.

On introduit ensuite l'ampoule ouverte dans un autoclave (Hastelloy C) de 125 ml. On purge alors deux fois l'autoclave par de l'azote à une pression de 15 bar, puis deux fois par de l'hydrogène à 10 bar.

On introduit ensuite dans l'autoclave 20 bars d'hydrogène, et on chauffe pendant 4 heures à 100 °C, et ceci sous agitation. En fin de réaction, on refroidit l'autoclave, on retire l'ampoule de verre, le carbure de tungstène décante et on soutire la phase organique.

Une analyse par dosage CPG (chromatographie en phase gazeuse) avec étalon interne donne les résultats suivants :

| | |
|---|---|
| | TT = 83 % |
| Bromo-6 naphtol-2 : | RR = 73 % |
| Bromo-6 naphtol-2 acétate : | RR = 6 % |
| Bromo-1 naphtol-2 : | RR = 3 % |

**EXEMPLE 2** : utilisation de WC + Cuivre

Dans une ampoule en verre de 35 ml, on introduit : 1,2 g de dibromo-1,6 naphtol-2 ; 40 mg de WC en poudre ; 12 mg de cuivre métallique ; et 15 ml d'acide acétique.

On procède ensuite comme à l'exemple 1.

L'analyse CPG donne les résultats suivants :

| | |
|---|---|
| | TT = 98 % |
| Bromo-6 naphtol-2 : | RR = 79 % |
| Bromo-1 naphtol-2 : | RR = 4 % |
| Bromo-6 naphtol-2 acétate : | RR = 8 % |

**EXEMPLE 3** : utilisation WC + Fer

Dans un SOTELEM de 300 ml, on introduit : 20 g de dibromo-1,6 naphtol-2 ; 1,29 g d'une poudre de WC ; 0,184 g de fer métallique en poudre ; et 100 ml d'acide acétique.

On procède ensuite comme à l'exemple 1, sauf que le chauffage à 100 °C n'est maintenu que pendant 1 heure 30.

L'analyse CPG donne les résultats suivants :

| | |
|---|---|
| | TT = 97 % |
| Bromo-6 naphtol-2 : | RT = 81 % |
| Bromo-6 naphtol-2 acétate : | RR = 13 % |
| Bromo-1 naphtol-2 : | RR = 2 % |

**EXEMPLE 4** : utilisation de WC seul

Dans un SOTELEM de 300 ml, on introduit : 100 ml de chlorobenzène ; 4 g d'acide bromhydrique ; 30 g de dibromo-1,6 naphtol-2 ; et 9,7 g de WC en poudre.

On procède ensuite comme à l'exemple 1, sauf que le chauffage à 100 °C n'est maintenu que pendant 3 heures.

L'analyse CPG donne les résultats suivants :

| | |
|---|---|
| Bromo-6 naphtol-2 : | TT = 100 %<br>RR = 96 % |

**EXEMPLE 5** : utilisation de WC seul

Dans un réacteur en HASTELLOY® de 300ml, on introduit : 100ml d'oxyde d'isopropyle ; 1,6 g d'acide bromhydrique ; 30 g de dibromo-1,6 naphtol-2 ; et 4,8 g de WC en poudre. On purge alors deux fois le réacteur par de l'azote à une pression de 10 bar, puis trois fois par de l'hydrogène à 10 bar. On chauffe à 120°C pendant 6 heures sous 20 bar d'hydrogéne, en maintenant le milieu sous agitation.

L'analyse CPG donne les résultats suivants :

| | |
|---|---|
| Bromo-6 naphtol-2 : | TT = 87 %<br>RR = 83 % |

**EXEMPLE 6**

Dans une ampoule en verre de 35 ml, on introduit : 1,5 g de dibromo-1,6 naphtol-2 ; 10 ml d'acétate d'éthyle ; 0,2 g d'acide bromhydrique ; et 0,48 g de WC en poudre.

On procéde ensuite comme à l'exemple 1.

L'analyse CPG donne ls résultats suivants :

| | |
|---|---|
| Bromo-6 naphtol-2 : | TT = 23 %<br>RR = 90 % |

**EXEMPLE 7** : utilisation d'un mélange de carbures (WC + VC)

Dans une ampoule en verre de 35 ml, on introduit : 1,5 g de dibromo-1,6 naphtol-2 ; 10 ml de toluéne ; 0,3 g d'acide bromhydrique ; et 0,48 g de carbure mixte de tungstène et de vanadium en poudre ( le vanadium représente 0,26 % du poids total des carbures).

On introduit ensuite l'ampoule ouverte dans un autoclave en Hastelloy HB2® de 125 ml. On purge alors deux fois l'autoclave par de l'azote à une pression de 15 bar, puis 2 fois avec 2 fois 10 bar d'hydrogène.

On place ensuite l'autoclave sous 20 bar d'hydrogène et on maintient cette pression pendant toute la durée de la réaction, ceci sous agitation et en maintenant la température à 120°C. Après 4 heures de réaction, on retire l'ampoule de verre, le carbure est décanté ou filtré.

On soutire la phase organique.

L'analyse CPG donne les résultats suivants :

| | |
|---|---|
| Bromo-6 naphtol-2 : | TT = 89 %<br>RR = 86 % |

## Revendications

1. Procédé de débromation de dérivés dibromés du naphtol, caractérisé par le fait que l'on fait réagir, dans un solvant organique acide et en présence d'un catalyseur à base de carbure de tungstène, (i) un dérivé dibromé du naphtol de formule :

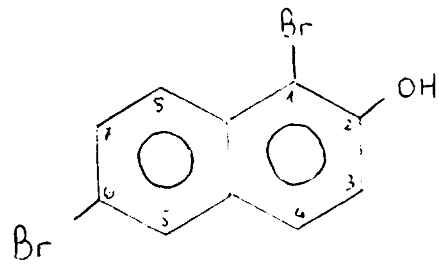

avec (ii) de l'hydrogène moléculaire ou un composé susceptible de générer, dans le milieu de réaction, de l'hydrogène naissant.

**2.** Procédé selon la revendication 1, caractérisé en ce que le catalyseur comprend un autre ou d'autres carbures de métal.

**3.** Procédé selon l'une des revendications 1 et 2, caractérisé en ce que ledit catalyseur se présente sous une forme divisée, en particulier sous la forme d'une poudre.

**4.** Procédé selon l'une des revendications 1 à 3, caractérisé en ce que la surface spécifique dudit catalyseur est comprise entre 1 et 400 $m^2/g$.

**5.** Procédé selon l'une quelconque des revendications précédentes, caractérisé en ce que l'on met en outre en oeuvre du fer et/ou du cuivre.

**6.** Procédé selon la revendication 5, caractérisé en ce que la quantité, en mole, de fer et/ou de cuivre mise en oeuvre est comprise entre 0,01 et 0,1 fois la quantité en mole de dérivé dibromé de départ.

**7.** Procédé selon l'une quelconque des revendications précédentes, caractérisé en ce que l'on utilise de l'hydrogène moléculaire $H_2$.

**8.** Procédé selon l'une quelconque des revendications 1 à 6, caractérisé en ce que ledit composé générant de l'hydrogène naissant est l'acide formique ou un formiate.

**9.** Procédé selon l'une quelconque des revendications précédentes, caractérisé en ce que l'on travaille sous pression.

**10.** Procédé selon l'une quelconque des revendications précédentes, caractérisé en ce qu'il est mis en oeuvre sur le produit résultant de la bromation directe du naphtol-2.

**11.** Procédé selon l'une quelconque des revendications précédentes, caractérisé en ce que ledit solvant organique acide est un acide carboxylique ou polycarboxylique, simple ou fonctionnalisé.

**12.** Procédé selon l'une des revendications 1 à 10, caractérisé en ce que ledit solvant organique acide est un solvant organique aprotique contenant un acide organique ou inorganique.

**13.** Procédé selon la revendication 12, caractérisé en ce que ledit solvant organique aprotique est choisi parmi les hydrocarbures aromatiques et les hydrocarbures halogénés, les éthers et les esters.

**14.** Utilisation du carbure de tungstène comme catalyseur d'hydrodébromation de dérivés dibromés du naphtol.

## Claims

**1.** Process for the debromination of dibrominated naphthol derivatives, characterised in that (i) a dibrominated naphthol derivative of formula:

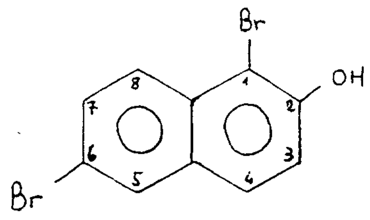

is reacted with (ii) molecular hydrogen or a compound capable of generating nascent hydrogen in the reaction mixture, in an acidic organic solvent and in the presence of a catalyst based on tungsten carbide.

2. Process according to Claim 1, characterised in that the catalyst comprises one or more other metal carbides.

3. Process according to either of Claims 1 and 2, characterised in that the said catalyst is in a divided form, in particular in the form of a powder.

4. Process according to one of Claims 1 to 3, characterised in that the specific surface area of the said catalyst is between 1 and 400 $m^2/g$.

5. Process according to any one of the preceding claims, characterised in that iron and/or copper are additionally used.

6. Process according to Claim 5, characterised in that the quantity, in moles, of iron and/or copper used is between 0.01 and 0.1 times the quantity in moles of starting dibrominated derivative.

7. Process according to any one of the preceding claims, characterised in that molecular hydrogen $H_2$ is used.

8. Process according to any one of Claims 1 to 6, characterised in that the said compound generating nascent hydrogen is formic acid or a formate.

9. Process according to any one of the preceding claims, characterised in that the work is carried out under pressure.

10. Process according to any one of the preceding claims, characterised in that it is used on the product resulting from the direct bromination of 2-naphthol.

11. Process according to any one of the preceding claims, characterised in that the said acidic organic solvent is a simple or functionalised carboxylic or polycarboxylic acid.

12. Process according to one of Claims 1 to 10, characterised in that the said acidic organic solvent is an aprotic organic solvent containing an organic or inorganic acid.

13. Process according to Claim 12, characterised in that the said aprotic organic solvent is chosen from aromatic hydrocarbons and halogenated hydrocarbons, ethers and esters.

14. Use of tungsten carbide as a catalyst for the hydrodebromination of dibrominated naphthol derivatives.

**Patentansprüche**

1. Verfahren zur Debromierung von Dibromnaphtholderivaten, dadurch gekennzeichnet, daß man (i) ein Dibromnaphtholderivat der Formel:

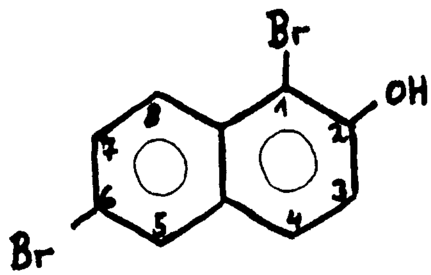

mit (ii) molekularem Wasserstoff oder einer Verbindung, die unter den Reaktionsbedingungen naszierenden Wasserstoff erzeugen kann, in einem sauren organischen Lösungsmittel und in Anwesenheit eines Katalysators auf Basis von Wolframcarbid reagieren läßt.

**2.** Verfahren gemäß Anspruch 1, dadurch gekennzeichnet, daß der Katalysator zusätzlich ein anderes oder weitere Metallcarbide enthält.

**3.** Verfahren gemäß einem der Ansprüche 1 oder 2, dadurch gekennzeichnet, daß der Katalysator in zerteilter Form, insbesondere in Form eines Pulvers, vorliegt.

**4.** Verfahren gemäß einem der Ansprüche 1 bis 3, dadurch gekennzeichnet, daß die spezifische Oberfläche des Katalysators zwischen 1 und 400 $m^2/g$ beträgt.

**5.** Verfahren gemäß einem der vorstehenden Ansprüche, dadurch gekennzeichnet, daß außerdem Eisen und/oder Kupfer zugesetzt werden.

**6.** Verfahren gemäß Anspruch 5, dadurch gekennzeichnet, daß die eingesetzte Molmenge an Eisen und/oder Kupfer zwischen dem 0,01 und dem 0,1fachen der Ausgangsmolmenge des Dibromderivates liegt.

**7.** Verfahren gemäß einem der vorstehenden Ansprüche, dadurch gekennzeichnet, daß man molekularen Wasserstoff $H_2$, verwendet.

**8.** Verfahren gemäß einem der Ansprüche 1 bis 6, dadurch gekennzeichnet, daß die naszierenden Wasserstoff erzeugende Verbindung Ameisensäure oder ein Formiat ist.

**9.** Verfahren gemäß einem der vorstehenden Ansprüche, dadurch gekennzeichnet, daß unter Druck gearbeitet wird.

**10.** Verfahren gemäß einem der vorstehenden Ansprüche, dadurch gekennzeichnet, daß es mit dem aus der direkten Bromierung von 2-Naphthol stammenden Produkt durchgeführt wird.

**11.** Verfahren gemäß einem der vorstehenden Ansprüche, dadurch gekennzeichnet, daß das saure organische Lösungsmittel eine einfache oder funktionelle Gruppen aufweisende Carbonsäure oder eine Polycarbonsäure darstellt.

**12.** Verfahren gemäß einem der Ansprüche 1 bis 10, dadurch gekennzeichnet, daß das saure organische Lösungsmittel ein aprotisches organisches Lösungsmittel darstellt, welches eine organische oder anorganische Säure enthält.

**13.** Verfahren gemäß Anspruch 12, dadurch gekennzeichnet, daß das aprotische organische Lösungsmittel aus aromatischen und Halogenkohlenwasserstoffen, Ethern und Estern ausgewählt ist.

**14.** Verwendung von Wolframcarbid als Katalysator zur Hydrodebromierung von Dibromnaphtholderivaten.